# EUROPEAN PATENT SPECIFICATION

(11) **EP 3 886 791 B1**
(45) Date of publication and mention of the grant of the patent: **12.11.2025**
(21) Application number: 19842581.1
(22) Date of filing: 28.11.2019
(51) Int. Cl.: A61K 8/26, A61K 8/37, A61Q 19/00, A61K 8/92, A61K 8/02

(54) **CLAY MASK COMPOSITION AND METHOD FOR USING THE SAME**
TONMASKENZUSAMMENSETZUNG UND VERFAHREN ZUR VERWENDUNG DAVON
COMPOSITION DE MASQUE D'ARGILE ET SON PROCÉDÉ D'UTILISATION

(30) Priority: 29.11.2018 EP 18209132
(43) Date of publication of application: 06.10.2021
(73) Proprietor: UNILEVER GLOBAL IP LIMITED, Wirral Merseyside CH62 4ZD (GB); Unilever IP Holdings B.V., 6708 WH Wageningen (NL)
(72) Inventor: TOMASHEVSKAIA, Marina, Trumbull, CT Connecticut 06611 (US); KEMLER, Kayla, Marie, Trumbull, CT Connecticut 06611 (US)
(74) Representative: James, Helen Sarah
(86) International application number: PCT/EP2019/082936
(87) International publication number: WO 2020/109481

(56) References cited:
- EP-A1- 0 576 287
- EP-A1- 2 067 409
- EP-A1- 2 735 340
- EP-B1- 2 067 409
- WO-A1-2006/089692
- WO-A1-2008/020497
- CA-A1- 2 949 527
- US-A- 4 640 932
- US-A- 5 599 546
- US-A1- 2006 239 955
- US-A1- 2009 130 220

## Description

### Field of the invention

The present invention is directed to a cosmetic composition, particularly a cosmetic clay mask composition. More particularly, the invention is directed to a clay mask composition and a method for using the same whereby the clay mask composition is substantially free of surfactant. The clay mask composition of the present invention surprisingly dries rapidly and simultaneously reduces negative sensory attributes of a typical clay mask, and results in excellent sensory benefits by eliminating a dry and tightening sensation on skin after product has been removed. The cosmetic clay mask composition of the present invention is excellent for sensitive skin since it is substantially free of surfactant and, surprisingly, is stable, displaying no syneresis or clumping after being stored at 45°C for 1 to 3.5 months.

### Background of the invention

Physical appearance, especially as it relates to skin characteristics, is a priority for many consumers. Consumers are not only interested in the latest fashions, but they are also very interested in the newest skin benefit technologies, including those that moisturize and reduce wrinkles on skin.

Creams, lotions, sprays and balms are some of the few formats used to provide benefit agents to skin. Additionally, cosmetic masks are known to offer a desirable delivery system for skin benefit agents. In fact, cosmetic masks are known to enhance the contact time of skin benefit agent to skin.

While cosmetic masks have positive attributes, the same are known to impart a dry and cracking sensation to skin after they are removed. It is of increasing interest to deliver a stable clay mask composition that dries rapidly, delivers skin benefit agents and yet does not impart a dry and cracking sensation to skin after being removed.

This invention, therefore, is directed to a cosmetic clay mask composition and a method for using the same. The clay mask composition comprises oil, water, thickening agent, and is substantially free of surfactant. Such a composition surprisingly dries rapidly, is stable and simultaneously results in excellent sensory benefits by not creating a dry and cracking sensation to skin after being removed.

### Additional Information

Efforts have been disclosed for making cosmetic masks. In U.S. Published Application No. 2004/0161435A1, skin firming and anti-aging cosmetic mask compositions are described.

Other efforts have been disclosed for making facial masks. In U.S. Patent No. 4,640,932, facial masks containing benzoyl peroxide are described.

Still other efforts have been disclosed for making facial masks. In U.S. Patent No. 9,333,164 B1, cosmetic compositions with pomace olive oil are described.

None of the additional information describes a clay mask composition as claimed, and particularly, one comprising oil, water and a thickening agent whereby the composition is substantially free of surfactant.

### Summary of the invention

In a first embodiment, the invention is directed to a cosmetic clay mask composition according to claim 1.

In a second embodiment, the present invention is directed to a non-therapeutic method for treating skin with the composition of the first embodiment of the invention.

Skin as used herein means skin on the feet, face, neck, chest, arms (including underarms), hands, legs, buttocks, back and scalp. The clay mask composition is preferably, however, applied to skin on the face. Clay, as used herein, means comprising kaolin and/or bentonite. Mask, as used herein, means a paste-like material having a viscosity from 150,000 to 375,000 mPa.s (cps) (determined using a Brookfield Helipath TD, 5 RPM, 1 minute, 25°C) and suitable for topical application and where paste-like means not pourable, but malleable. Such a mask can be applied to any skin on the body whereby skin on the face is often desired. Dries rapidly means drying within 20 minutes (in another embodiment within 12 minutes) after being applied to skin at a thickness of 0.1 to 0.75 millimeters at 25°C. Dry and cracking sensation means having tight (e.g., dry, draggy, sticky) skin that feels dehydrated after washing off mask composition. Substantially free of surfactant means less than 0.1% by weight of the total weight of mask composition, and yet in another embodiment, means less than 0.07% by weight of the composition. In still another embodiment, substantially free of surfactant means from 0.001 to 0.01% by weight surfactant based on total weight of the mask composition. In still another embodiment, the clay mask composition of the present invention comprises no (0.0% by weight) surfactant. Kaolin (or kaolinite) means a colloidal clay mineral having a chemical composition comprising Al₂Si₂O₅(OH)₄. Such a mineral is suitable to adhere to an oil and water interface of the clay mask composition and may induce formation of a pickering emulsion. Bentonite, as used herein, means comprising at least 75% by weight smectite (i.e., montmorillonite) based on total weight of the bentonite. Stable as used herein means displaying no syneresis (separation) or clumping after being stored at room temperature, 45°C for 1 to 3.5 months and 4°C for 1 to 3.5 months. Room temperature means 25°C. In another embodiment, stable means displaying no syneresis or clumping after storage at room temperature, at 45°C for 2 to 3 months, and 4°C for 2 to 3 months. Stable also means no syneresis or clumping after three (3) storage cycles at -8°C to 25°C in a twenty-three (23) hour period for each cycle (Stability Protocol).

For the avoidance of doubt, and by illustration, a composition comprising kaolin, water and bentonite is meant to include a composition consisting essentially of and consisting of the same. All percentages used herein are meant to be by weight based on total weight of final composition unless stated otherwise. All ranges identified herein are meant to include all ranges subsumed therein if, for example, reference to the same is explicitly made. Except in the Examples, or where otherwise explicitly indicated, all numbers in this description indicating amounts of material or conditions of reaction, physical properties of materials and/or use are to be understood as modified by the word "about".

### Detailed Description of the Invention

The only limitation with respect to the bentonite used in the present invention is that the same is suitable for use for topical application in a consumer product. The bentonite is at least 75% by weight smectite and in still another embodiment, the bentonite used in this invention is from 80 to 90% by weight smectite (Bentonite comprising 63 to 73% by weight SiO₂ and 17 to 24% by weight Al₂O₃).

The bentonite suitable for use in the clay mask composition of the present invention is made commercially available from suppliers like H&H Clay, Inc., Wyoming Bentonite, Brenntag Specialties, Inc and Sigma Aldrich. Use of bentonite made available from Brenntag Specialties, Inc under the Bentonite NF BC-670 name (CAS No. 1302-78-9) is often preferred.

Bentonite makes up from 5 to 30% by weight of the clay mask composition. In another embodiment, bentonite makes up from 10 to 25% by weight of the clay mask composition, and still in another embodiment the clay mask composition comprises 15 to 20% by weight bentonite, based on total weight of the clay mask composition and including all ranges subsumed therein.

The bentonite used in the present invention is provided with (i.e., pre-mixed, agglomerated and/or chemically bonded with) a thickener selected from xanthan gum, citrus fiber, guar gum, pectin, tapioca starch, hydroxypropyl cellulose, locust bean gum or a mixture thereof. The thickener makes up from 2 to 8% by weight of the total weight of the bentonite and thickener combination, including all ranges subsumed therein. In another embodiment, the total weight of thickener provided with bentonite and thickener is from 3 to 7%, and still in another embodiment from 3.5 to 6.0% by weight, based on total weight of bentonite and thickener and including all ranges subsumed therein Preferred is thickener that comprises at least 50% by weight, and preferably, 60 to 100% by weight, and most preferably, 90 to 100% by weight xanthan gum, based on total weight of thickener provided with bentonite and including all ranges subsumed therein. In still another embodiment, the thickener provided with bentonite is 100% by weight xanthan gum. The same is commercially sold by suppliers like, for example, Ephyla under the Frametime CX name.

In still another embodiment, additionall thickener is used in the cosmetic mask composition but is added as an ingredient and not provided with bentonite. When used, additional , thickener added as an ingredient often makes up from 0.10 to 0.5%, typically from 0.12 to 0.4%, and in another embodiment from 0.15 to 0.35% by weight of the clay mask composition, including all ranges subsumed therein. Similar to the thickener provided with bentonite, the additional thickener added as an ingredient is preferably at least 50% by weight, and preferably, 60 to 100% by weight, and most preferably, 90 to 100% by weight xanthan gum, based on total weight of thickener added as an ingredient and including all ranges subsumed therein. In still another embodiment, the thickener added as an ingredient is 100% by weight xanthan gum.

The oil suitable for use in this invention is limited only to the extent that the same is suitable for use in a topical skin application. Illustrative examples of the oils suitable for use in this invention include mineral oil, isopropyl myristate, octylhydroxystearate, isopropyl palmitate, conjugated linoleic acid, petroselenic acid, shea butter, silicone oil, squalene, petrolatum, docosahexaenoic acid (DHA) and eicosopentaenoic (EPA), algal oil, flaxseed oil, and triglycerides like caprylic capric triglyceride, grapeseed oil, meadowfoam oil, linseed oil, rye oil, safflower oil, red kuri oil, sesame oil, rapeseed oil, primrose oil, millet oil, barley oil, passion flower oil, mixtures thereof or the like.

In one embodiment, caprylic capric triglyceride is the oil of choice. In another embodiment, oil makes up from 2 to 25% by weight, and in another preferred embodiment 3 to 18% by weight of the total weight of the clay mask composition, including all ranges subsumed therein. In still another embodiment, oil makes up from 4 to 12% by weight of the clay mask composition, based on total weight of the clay mask composition and including all ranges subsumed therein. In even another embodiment, oil makes up from 3 to 7% by weight of the composition based on total weight of the clay mask composition and including all ranges subsumed therein. In still another embodiment caprylic capric triglyceride and isopropyl myristate are used together, with the combination being predominately caprylic capric triglyceride (55 to 95%, preferably 65 to 90%, and most preferably, 65 to 85% by weight caprylic capric triglyceride based on total weight of caprylic capric triglyceride and isopropyl myristate.

The clay composition of the present invention will comprise water. Typically, water makes up from 20 to 80% by weight of the clay mask composition, based on total weight of the clay mask composition and including all ranges subsumed therein. In another embodiment, water makes up from 25 to 75% by weight and yet in another preferred embodiment water makes up from 45 to 70% by weight of the total weight of the clay mask composition and including all ranges subsumed therein.

The kaolin suitable for use in this invention is a colloidal (particles from 1 to 1000 nanometers) clay mineral, again, having a chemical composition comprising Al₂Si₂O₅(OH)₄. Such a mineral is suitable to adhere to the oil and water interface of the clay mask composition. Typically, the colloidal kaolin will comprise 40 to 50% by weight SiO₂, 35 to 45% by weight Al₂O₃ and 1 to 2.5% by weight TiO₂. Kaolin typically makes up from 2 to 35% by weight of the clay mask composition. In one embodiment, Kaolin makes up from 3 to 30% by weight and in another embodiment from 10 to 25% by weight of the clay mask composition and including all ranges subsumed therein.

The clay mask composition of the present invention is typically prepared by mixing the ingredients with moderate shear (typically with a propeller blade) under atmospheric conditions and ambient temperature. Heating is optional if solid ingredients are present. Mixing is stopped subsequent to obtaining a homogeneous composition which preferably is homogenized using a Silverson Homogenizer until a clay mask composition having a viscosity from 150,000 to 375,000 Pa.s (cps) is recovered. When heating is desired, the temperature of the composition is raised from ambient to 35 to 50°C, including all ranges subsumed therein.

The viscosity of the clay mask composition is from 150,000 to 375,000 Pa.s (cps) (determined using a Brookfield Helipath TD, 5 RPM, 1 minute, 25°C). In another embodiment, the viscosity of the clay mask composition is from 175,000 to 285,000 Pa.s (cps), and in still another embodiment, from 200,000 Pa.s (cps) to 275,000 Pa.s (cps), including all ranges subsumed therein.

The clay mask composition of the present invention, as defined, comprises less than 0.1% by weight surfactant based on total weight of the clay mask composition is present. In another embodiment, less than 0.07% by weight and in still another embodiment 0.001 to 0.01% by weight surfactant is used. In yet another embodiment, no (0.0% by weight) surfactant or emulsifier is used.

If surfactant is optionally desired, illustrative examples include lecithin, sorbitan laurate, glyceryl stearate, laureth-4, mixtures thereof or the like. Other surfactants having an HLB of 6 to 9 may be used as long as such surfactants are suitable for use in a consumer product.

Optional ingredients suitable for use in the clay mask composition of the present invention include skin benefit agents. Illustrative hydrophilic benefit agents (suitable for addition to water in the clay mask composition) include 4-ethyl resorcinol, extracts like chamomile, green tea, liquorice, grape seed, sage, aloe vera, thyme, rosemary mixtures thereof or the like. Other hydrophilic benefit agents include humectants like glycerine and/or sorbitol, vitamins such as vitamin C and vitamin B3 (niacinamide), as well as dihydroxyacetone, and sunscreens like benzophenone-4 and phenylbenzimidazole sulphonic acid, mixtures thereof or the like. When used, hydrophilic benefit agent makes up from 0.1-12% by weight of the clay mask composition. In another embodiment the hydrophilic benefit agent makes up from 1-10% by weight and in still another embodiment from 2-8% by weight of the clay mask composition, including all ranges subsumed therein. Inclusion of humectant, like sorbitol and/or glycerin is often desired. Yet in another embodiment, the clay mask composition comprises in its water phase 2 to 7% by weight of glycerine, sorbitol or both.

Other optional benefit agents include hydrophobic agents. Illustrative examples of the hydrophobic agents suitable for addition to oil in the clay mask composition of the present invention include vitamins like Vitamin A (and its oil soluble derivatives), D, E (and its oil soluble derivatives) and K, sunscreens like methoxycinnamate, octyl methoxycinnamate, bis-ethyl hexyloxyphenol methoxyphenyl triazine, drometrizole trisiloxane or mixtures thereof. Even other hydrophobic agents suitable for inclusion into the oil in the clay mask composition include thymol, terpineol, hydroxyacids, resorcinols like 4-hexyl resorcinol, 4-phenylethyl resorcinol, 4-cyclopentyl resorcinol, 4-cyclohexyl resorcinol, mixtures thereof or the like. When employed, hydrophobic agent makes up from 0.001 to 3.5% by weight of the clay mask composition. In another embodiment 0.01 to 3% by weight and still another embodiment from 0.5 to 2% by weight of the clay mask composition of the present invention, including all ranges subsumed therein.

Preservatives can desirably be incorporated into the clay mask compositions of this invention to protect against the growth of potentially harmful microorganisms. Suitable traditional preservatives for use in this invention are alkyl esters of para-hydroxybenzoic acid. Other preservatives include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate and benzyl alcohol. The preservatives should be selected having regard for the use of the composition and possible incompatibilities between the preservatives and other ingredients in the composition. Preservatives are preferably employed in amounts ranging from 0.01% to 2.5% by weight of the total weight of the clay mask composition, including all ranges subsumed therein. Combinations of 1,2-octanediol and phenoxyethanol, or iodopropynyl butyl carbamate and phenoxyethanol are preferred, with phenoxyethanol and 1,2-octanediol, collectively and preferably, making up less than 2% by weight of the total weight of the clay mask composition of the present invention. Also preferred is a preservative system with hydroxyacetophenone alone or in a mixture with other preservatives.

In order to stabilize certain oils and benefit agents, it is within the scope of the present invention to use antioxidants that are suitable for cosmetic compositions. Illustrative example of the antioxidants suitable for use in the present invention are Tinogard DA, Tinogard TT (commercially available from BASF), butylated hydroxytoluene, propyl gallate, tert-butyl hydoquinone mixture thereof or the like. When used, antioxidant makes up from about 0.001 to 1.0% by weight of the total weight of the cosmetic mask composition. In another embodiment, antioxidant makes up from 0.01 to 0.5% by weight and yet in another embodiment from 0.02 to 0.1% by weight of the total weight of the cosmetic mask composition, including all ranges subsumed therein.

Conventional packaging such as jars, tubes and sachets may be used to package the cosmetic clay compositions of this invention.

The following examples are provided to facilitate an understanding of the present invention. The examples are not intended to limit the scope of the claimed invention.

### Example 1

The Samples below were prepared by mixing all ingredients in a mixing vessel under moderate shear with an electric driven steel propeller blade. The resulting mask composition was homogenized using a Silverson Homogenizer until a product having a viscosity of about 250,000 cps was recovered.

**TABLE I**

| | **Samples** | | | | | | | | | | |
|---|---|---|---|---|---|---|---|---|---|---|---|
| **Ingredient** | **A** | **B** | **C** | **D** | **E** | **F** | **G** | **H** | **I** | **J** | **K** |
| Water | 68.4 | 58.4 | 63.4 | 46.9 | 69.4 | 61.1 | 61.1 | 46.1 | 63.4 | 71.1 | 61.1 |
| Glycerin | 3 | 3 | 3 | 0 | 0 | 3 | 3 | 3 | 3 | 3 | 3 |
| Caprylic/Capric Triglycerides | 4 | 4 | 8 | 17.5 | 0 | 4 | 4 | 20 | 8 | 8 | 16 |
| Isopropyl Myristate | 1 | 1 | 2 | 10 | 0 | 1 | 1 | 0 | 2 | 2 | 4 |
| Preservatives | 0.6 | 0.6 | 0.6 | 0.6 | 0.6 | 0.6 | 0.6 | 0.6 | 0.6 | 0.6 | 0.6 |
| Bentonite* | 15 | 10 | 15 | 7.5 | 10 | 10 | 10 | 10 | 20 | 15 | 15 |
| Kaolin | 5 | 20 | 5 | 10 | 20 | 20 | 20 | 20 | 0 | 0 | 0 |
| Xanthan Gum | 0 | 0 | 0 | 0 | 0 | 0.3 | 0.3 | 0.3 | 0 | 0.3 | 0.3 |
| Frametime CX ** | 3 | 3 | 3 | 7.5 | 0 | 0 | 0 | 0 | 3 | 0 | 0 |

| | | | | | | | | | | | |
|---|---|---|---|---|---|---|---|---|---|---|---|
| * greater than 80% by weight smectite by weight ** bentonite and 5% by weight xanthan gum | | | | | | | | | | | |

All samples were stored according to the described Stability Protocol. Samples A through D and F through H (made according to the present invention) unexpectedly did not display syneresis or clumping after storage. Such clay mask compositions were homogeneous after storage, notwithstanding the fact that surfactant was not used in the compositions with significant levels of water and oil.

Sample E, which was free of oil and xanthan gum, was clumpy and unusable after storage. Samples I through K, not made consistent with this invention, displayed phase separation after storage.

**TABLE II**

| | **Samples** | | | | | | | |
|---|---|---|---|---|---|---|---|---|
| **Ingredient** | **Mineral Oil** | **Sunflower Seed Oil** | **Conjugated Linoleic Acid** | **Meadowfoam Seed Oil** | **Dimethicone 50cst** | **Isopropyl palmitate** | **Linseed Oil** | **Octyl Methoxycinnamate** |
| Water | 68.4 | 68.4 | 68.4 | 68.4 | 68.4 | 68.4 | 68.4 | 68.4 |
| Glycerin | 3 | 3 | 3 | 3 | 3 | 3 | 3 | 3 |
| Oil* | 5 | 5 | 5 | 5 | 5 | 5 | 5 | 5 |
| Preservative | 0.6 | 0.6 | 0.6 | 0.6 | 0.6 | 0.6 | 0.6 | 0.6 |
| Bentonite** | 15 | 15 | 15 | 15 | 15 | 15 | 15 | 15 |
| Kaolin | 5 | 5 | 5 | 5 | 5 | 5 | 5 | 5 |
| Frametime CX *** | 3 | 3 | 3 | 3 | 3 | 3 | 3 | 3 |
| AOX | 0.05 | 0.05 | 0.05 | 0.05 | 0.05 | 0.05 | 0.05 | 0.05 |

| | | | | | | | | |
|---|---|---|---|---|---|---|---|---|
| * as identified at top of each column ** greater than 80% by weight smectite by weight *** bentonite and 5% by weight xanthan gum | | | | | | | | |

All samples were stored according to the described Stability Protocol. The clay mask compositions were made according to this invention and surprisingly displayed no syneresis or clumping after storage, notwithstanding the fact that surfactant was not used in compositions with significant levels of water and oil.

**TABLE III**

| | **Samples** | | | | |
|---|---|---|---|---|---|
| **Ingredient** | **Grapeseed Oil/Isopropyl Myristate** | **Octylhydroxy Stearate/ Dimethicone 50 cst** | **Conjugated linoleic/Caprylic/Capric Triglyceride** | **Meadowfoam Seed Oil/Isopropyl Palmitate** | **Sunflower Seed Oil/Octylmethoxy cinnamate** |
| **Ratio of oil by weight, respectively** | **60/40** | **20/80** | **10/90** | **70/30** | **40/60** |
| Water | 68.4 | 68.4 | 68.4 | 68.4 | 68.4 |
| Glycerin | 3 | 3 | 3 | 3 | 3 |
| Oil* | 5 | 5 | 5 | 5 | 5 |
| Preservative | 0.6 | 0.6 | 0.6 | 0.6 | 0.6 |
| Bentonite** | 15 | 15 | 15 | 15 | 15 |
| Kaolin | 5 | 5 | 5 | 5 | 5 |
| Xanthan Gum | 0 | 0 | 0 | 0 | 0 |
| Frametime CX *** | 3 | 3 | 3 | 3 | 3 |
| Antioxidant | 0.05 | 0.05 | 0.05 | 0.05 | 0.05 |

| | | | | | |
|---|---|---|---|---|---|
| * as identified at top of each column ** greater than 80% smectite by weight *** bentonite and 5% by weight xanthan gum | | | | | |

All samples were stored according to the described Stability Protocol. The clay mask compositions were made according to this invention and surprisingly displayed no syneresis or clumping after storage, notwithstanding the fact that surfactant was not used in compositions with significant levels of water and oil.

### Example 3

The following Samples were tested for sensory characteristics by applying clay mask composition to skin on a portion of the forearms (about 3.75 cm x 5 cm) and hands of twelve (12) trained panelist. The compositions were allowed to dry (about 10 minutes after application) and subsequently washed off with warm water. Skin having been subjected to clay mask composition was assessed and the results below reflect the feedback from the number of panelists of the twelve in the study. The results in the study unexpectedly show that the majority of panelists using the clay mask composition of the present invention concluded that less skin attributes associated with tight and dehydrated skin tightening resulted when using the composition of the present invention. Moreover, it was unexpectedly shown that the compositions of the present invention have more favorable attributes than commercially available product with surfactant.

| | Sample E | Sample C | Commercial Clay Mask * |
|---|---|---|---|
| **Desirable** | | | |
| Soft | 1 | 7 | 6 |
| Silky | 1 | 7 | 1 |
| Smooth | 2 | 8 | 4 |

| **Not Desirable** | | | |
|---|---|---|---|
| Dry | 3 | 2 | 3 |
| Draggy | 10 | 4 | 8 |
| Greasy | 1 | 0 | 0 |
| Sticky | 5 | 0 | 3 |

| | | | |
|---|---|---|---|
| * Similar to commercially available L'Oreal Clay Mask composition with surfactant. | | | |

All numbers represent the number of panelists out of the 12 concluding the skin attribute was identifiable.

### Example 4

A Shimpo FGE-1XY Digital Force Gauge 500g/5N was used to measure frictional force of a bioskin plate after 3.5g of clay mask composition was applied. The bioskin plate (P001-001), supplied by Beaulax Co., Ltd., Japan, was used as a substrate to mimic a skin surface. A rectangular piece (weighed down to 25g) of bioskin plate (about 2.5 x 3.0 cm) was attached to a nylon string and via a mechanical rotating unit pulled constantly at 18 cm per minute. A digital force gauge was connected to the string in relation to the bioskin plate.

Each sample (product) tested was evenly applied to a 2.5 x 19 cm surface on the bioskin plate. After 10 min of drying time, product was washed off using 30°C tap water while gently rubbing to remove all residual cosmetic clay composition. The plate was blotted dry with paper towel. Friction was measured while dragging the load along the 19 cm path. Five (5) measurements were taken for each product, averaged and a standard deviation was found. The results are as follows:

| **Friction Force (g)** | **Baseline** | **C** | **E** | **F** | **Formula F with Tween 20 added (3wt%)** | **Neutroge na Clay Mask** | **Clay Mask L'Oreal** |
|---|---|---|---|---|---|---|---|
| Point 1 | 11.3 | 13.3 | 44.2 | 15.4 | 37.3 | 39.9 | 55.2 |
| Point 2 | 11.9 | 15.9 | 44.4 | 14.3 | 38.4 | 45.1 | 54.4 |
| Point 3 | 12.5 | 17.1 | 40.8 | 15.8 | 38.2 | 47 | 52.1 |
| Point 4 | 11.9 | 18.2 | 40.3 | 16.3 | 37.2 | 45.2 | 50 |
| Point 5 | 12.5 | 19.1 | 38.7 | 16 | 35.6 | 49.1 | 50.2 |
| Average | 12.0 ± 0.5 | 16.7 ± 2.0 | 41.7 ± 2.3 | 15.6 ± 2.3 | 37.3 ± 1.0 | 45.3 ± 3.1 | 52.4 ± 2.1 |

Samples C and F (with no surfactant, 10 and 5% oil, respectively) made consistent with the present invention correspond to smaller friction numbers (less surface roughness) after application compared to controls and commercial products with surfactant and without oil.

The results unexpectedly show that the formulas made according to the present invention had drying times consistent with conventional formulas with surfactant and no oil while simultaneously resulting in a sensory feel with less friction, and therefore, less of a dry and tightening sensation after being washed off. The data supports the conclusion that compositions of the present invention do not impart negative sensory attributes after use.

## Claims

1. A cosmetic clay mask composition comprising:
(1) from 5 to 30% by weight (based on the total weight of the clay mask composition) bentonite, the bentonite being at least 75% by weight smectite;
(2) kaolin;
(3) oil; and
(4) water; and
(5) thickener, in which the bentonite used is provided with (i.e. premixed, agglomerated and/or chemically bonded with) the thickener; the thickener making up from 2 to 8%, preferably from 3.0 to 7.0%, by weight of the total weightof the bentonite and thickener combination; and
the thickener being selected from xanthan gum, citrus fiber, guar gum, pectin, tapioca starch, hydroxypropyl cellulose, locust bean gum or a mixture thereof; and the thickener comprising at least 50%, preferably 90 to 100%, by weight xanthan gum,
the composition comprising less than 0.1% by weight surfactant based on total weight of the clay mask composition is present.

2. The composition according to any of the preceding claims wherein the composition has a viscosity of 150,000 to 375,000 mPa.s (cps) (determined using a Brookfield Helipath TD, 5 RPM, 1 minute, 25°C) and comprises 0.001 to 0.07% by weight surfactant.

3. The composition according to any of the preceding claims wherein the composition is free of surfactant.

4. The composition according to any of the preceding claims wherein the composition comprises a hydrophilic benefit agent, hydrophobic benefit agent or both.

5. The composition according to claim 6 wherein the hydrophilic benefit agent is 4-ethyl resorcinol, chamomile, green tea, liquorice, grape seed, sage, aloe vera, thyme, rosemary extract, glycerine, sorbitol, vitamin C, vitamin B3, dihydroxyacetone, benzophenone-4, phenylbenzimidazole sulphonic acid or a mixture thereof.

6. The composition according to any of the claims 6 to 7 wherein the hydrophobic benefit agent is Vitamin A, D, E or K, methoxycinnamate, octyl methoxycinnamate, bis-ethyl hexyloxyphenol methoxyphenyl triazine, drometrizole trisiloxane, a hydroxyacid, thymol, terpineol, 4-hexyl resorcinol, 4-phenylethyl resorcinol, 4-cyclopentyl resorcinol, 4-cyclohexyl resorcinol, or a mixture thereof.

7. A non-therapeutic method for treating skin with the composition according to any of the preceding claims.

## Patentansprüche

1. Kosmetische Tonmaskenzusammensetzung, umfassend:
(1) 5 bis 30 Gew.-% (bezogen auf das Gesamtgewicht der Tonmaskenzusammensetzung) Bentonit, wobei der Bentonit zu mindestens 75 Gew.-% aus Smectit besteht;
(2) Kaolin;
(3) Öl; und
(4) Wasser, und
(5) Verdickungsmittel,
wobei der verwendete Bentonit mit dem Verdickungsmittel versehen ist (d.h., vorgemischt, agglomeriert und/oder chemisch gebunden ist);
wobei das Verdickungsmittel von 2 bis 8 Gew.-%, vorzugsweise von 3,0 bis 7,0 Gew.-%, des Gesamtgewichts der Kombination aus Bentonit und Verdickungsmittel ausmacht; und
wobei das Verdickungsmittel unter Xanthangummi, Citrusfasern, Guarkernmehl, Pektin, Tapiokastärke, Hydroxypropylcellulose, Johannisbrotkernmehl oder einer Mischung davon ausgewählt ist und das Verdickungsmittel mindestens 50 Gew.-%, vorzugsweise 90 bis 100 Gew.-%, Xanthangummi umfasst,
wobei die Zusammensetzung weniger als 0,1 Gew.-% Tensid, bezogen auf das Gesamtgewicht der Tonmaskenzusammensetzung, umfasst.

2. Zusammensetzung nach einem der vorhergehenden Ansprüche, wobei die Zusammensetzung eine Viskosität von 150.000 bis 375.000 mPa.s (cps) (bestimmt unter Verwendung eines Brookfield Helipath TD, 5 U/min, 1 Minute, 25°C) aufweist und 0,001 bis 0,07 Gew.-% Tensid umfasst.

3. Zusammensetzung nach einem der vorhergehenden Ansprüche, wobei die Zusammensetzung frei von Tensid ist.

4. Zusammensetzung nach einem der vorhergehenden Ansprüche, wobei die Zusammensetzung ein hydrophiles Pflegemittel, ein hydrophobes Pflegemittel oder beides umfasst.

5. Zusammensetzung nach Anspruch 4, wobei das hydrophile Pflegemittel 4-Ethylresorcin, Kamille, grüner Tee, Süßholz, Traubenkern, Salbei, Aloe Vera, Thymian, Rosmarinextrakt, Glycerin, Sorbit, Vitamin C, Vitamin B3, Dihydroxyaceton, Benzophenon-4, Phenylbenzimidazolsulfonsäure oder eine Mischung davon ist.

6. Zusammensetzung nach einem der Ansprüche 4 bis 5, wobei das hydrophobe Pflegemittel Vitamin A, D, E oder K, Methoxycinnamat, Octylmethoxycinnamat, Bis-Ethylhexyloxyphenolmethoxyphenyltriazin, Drometrizol-Trisiloxan, eine Hydroxysäure, Thymol, Terpineol, 4-Hexylresorcin, 4-Phenylethylresorcin, 4-Cyclopentylresorcin, 4-Cyclohexylresorcin oder eine Mischung davon ist.

7. Nicht-therapeutisches Verfahren zur Behandlung der Haut mit der Zusammensetzung nach einem der vorhergehenden Ansprüche.

## Revendications

1. Composition de masque d'argile cosmétique comprenant :
(1) de 5 à 30 % en poids (en se basant sur le poids total de la composition de masque d'argile) de bentonite, la bentonite étant constituée d'au moins 75 % de smectite ;
(2) du kaolin ;
(3) une huile ; et
(4) de l'eau ; et
(5) un épaississant, dans lequel la bentonite utilisée est fournie avec (c.-à-d. prémélangée, agglomérée et/ou chimiquement liée avec) l'épaississant ; l'épaississant représentant de 2 à 8 %, de préférence de 3,0 à 7,0 %, en poids du poids total de la combinaison de bentonite et d'épaississant ; et
l'épaississant étant choisi parmi la gomme xanthane, une fibre d'agrume, la gomme de guar, la pectine, l'amidon de tapioca, l'hydroxypropylcellulose, la gomme de caroube ou un mélange de ceux-ci ; et l'épaississant comprenant au moins 50 %, de préférence de 90 à 100 % en poids de gomme xanthane,
la composition comprenant moins de 0,1 % en poids de tensioactif en se basant sur le poids total de la composition de masque d'argile est présente.

2. Composition selon l'une quelconque des revendications précédentes dans laquelle la composition a une viscosité de 150 000 à 375 000 mPa.s (cps) (déterminée en utilisation un Brookfield Helipath TD, 5 tr/min, 1 minute, 25 °C) et comprend de 0,001 à 0,07 % en poids de tensioactif.

3. Composition selon l'une quelconque des revendications précédentes dans laquelle la composition est exempte de tensioactif.

4. Composition selon l'une quelconque des revendications précédentes dans laquelle la composition comprend un agent bénéfique hydrophile, un agent bénéfique hydrophobe ou les deux.

5. Composition selon la revendication 6 dans laquelle l'agent bénéfique hydrophile est le 4-éthyl-résorcinol, la camomille, le thé vert, la réglisse, les pépins de raisin, la sauge, l'aloe vera, le thym, un extrait de romarin, la glycérine, le sorbitol, la vitamine C, la vitamine B3, la dihydroxyacétone, la benzophénone-4, l'acide phénylbenzimidazole sulfonique ou un mélange de ceux-ci.

6. Composition selon l'une quelconque des revendications 6 à 7 dans laquelle l'agent bénéfique hydrophobe est la vitamine A, D, E ou K, le méthoxycinnamate, le méthoxycinnamate d'octyle, la bis-éthyl-hexyloxyphénol-méthoxyphényl-triazine, le drométrizole trisiloxane, un hydroxyacide, le thymol, le terpinéol, le 4-hexyl-résorcinol, le 4-phényléthyl-résorcinol, le 4-cyclopentyl résorcinol, le 4-cyclohexyl-résorcinol, ou un mélange de ceux-ci.

7. Procédé non thérapeutique destiné au traitement de la peau avec la composition selon l'une quelconque des revendications précédentes.
